# EUROPÄISCHE PATENTSCHRIFT

(11) **EP 1 469 745 B1**
(45) Veröffentlichungstag und Bekanntmachung des Hinweises auf die Patenterteilung: **28.12.2011**
(21) Anmeldenummer: 03702387.6
(22) Anmeldetag: 07.01.2003
(51) Int. Cl.: A23L 1/275, C08K 7/00, C09B 67/00, A61K 47/48

(54) **VERWENDUNG VON MEHRSCHICHTPIGMENTEN IM LEBENSMITTEL UND PHARMABEREICH**
USE OF MULTI-LAYER PIGMENTS IN THE FOOD AND PHARMACEUTICAL INDUSTRY
UTILISATION DE PIGMENTS MULTICOUCHE DANS LE DOMAINE ALIMENTAIRE ET PHARMACEUTIQUE

(30) Priorität: 01.02.2002 DE 10204336
(43) Veröffentlichungstag der Anmeldung: 27.10.2004
(73) Patentinhaber: Merck Patent GmbH, 64293 Darmstadt (DE)
(72) Erfinder: SCHWEINFURTH, Ralf, 64287 Darmstadt (DE)
(86) Internationale Anmeldenummer: PCT/EP2003/000044
(87) Internationale Veröffentlichungsnummer: WO 2003/063616

(56) Entgegenhaltungen:
- EP-A- 1 045 014
- WO-A1-99/061529
- WO-A1-99/061529
- WO-A2-03/002149
- WO-A2-03/002149
- DE-A- 19 618 569
- DE-A- 19 638 708
- DE-A- 19 831 869
- US-A- 3 087 828
- US-A- 4 552 593
- US-B1- 6 334 893
- EM INDUSTRIES, INC. (NEW YORK) PETITION TO FDA OF APRIL 21, 1998
- EM INDUSTRIES, INC. (NEW YORK) PETITION TO FDA, JULY 20, 1998
- EM INDUSTRIES, INC. PETITION TO FDA 21 April 1998, NEW YORK,
- EM INDUSTRIES, INC. PETITION TO FDA 20 Juli 1998, NEW YORK,

## Beschreibung

Die vorliegende Erfindung betrifft die Verwendung von Mehrschichtpigmenten auf Basis plättchenförmiger Substrate zur Einfärbung von Lebensmittel- und Pharmaerzeugnissen.

Neben den funktionellen Anwendungen werden Mehrschichtpigmente und Pigmentmischungen, wie z.B. bekannt aus EP-A-1 045 014, US-B1-6 334 893 und WO 99/061529, auch immer mehr zur optischen Aufwertung von Produkten, z. B. in der Kosmetik, eingesetzt, da schöne Farben und Effekte beim Betrachter und Konsumenten angenehme subjektive Empfindungen hervorrufen. Mehrschichtpigmente, sofern sie unter den entsprechenden, strengen Reinheits- und Qualitätsbestimmungen hergestellt wurden, sollten auch im Nahrungsmittel- und Pharmabereich zur Verbesserung des Farbeffektes bzw. zur Farbgebung einsetzbar sein.

Die Verwendung von Perlglanz- bzw. Interferenzpigmenten zur Farbgebung von Produkten aus dem Lebensmittel- und Pharmabereich ist aus der DE 198 31 869 und WO 03/002149 A bekannt. Diese Produkte haben aber den Nachteil, dass die ursprüngliche Farbe des zu färbenden Erzeugnisses mehr oder weniger stark überdeckt wird. Oftmals ist es aber erwünscht, dass die Original-Produktfarbe erhalten bleibt und diese nur durch einen zusätzlichen Farb-Glanz-Effekt veredelt wird. Dadurch bedingt kann auch der Farbstoffanteil im zu färbenden Produkt vor der Färbung mit den Mehrschichtpigmenten verringert werden,

Aufgabe der vorliegenden Erfindung ist es daher die Palette der bereits bekannten Farbtöne bei der Einfärbung von Lebensmitteln- und Pharmaerzeugnissen zu erweitern, wodurch die Produkte eine zusätzliche sinnlich wahrnehmbare Aufwertung erfahren. Die optische Aufwertung ist insbesondere für Pharmaprodukte wertvoll, da eine deutlichere Differenzierung verschieden gefärbter Tabletten, Dragees, etc., ermöglicht wird.

Überraschenderweise wurde nun gefunden, dass zur optischen Aufwertung von Lebensmittel- und Pharmaerzeugnissen Mehrschichtpigmente auf Basis von plättchenförmigen Substraten beschichtet mit alternierenden Schichten von Metalloxiden hervorragend geeignet sind. Besonders bevorzugte Metalloxide sind TiO₂, Fe₂O₃ und Fe₃O₄. Insbesondere die Kombination von Mehrschichtpigmenten mit TiO₂- und/oder Fe₃O₄-Schichten mit natürlichen bzw. naturidentischen Farbstoffen, Farbpigmenten oder färbenden Frucht- und Pflanzenextrakten, verleihen dem Lebensmittelerzeugnis einen interessanten neuen Farbton. Gegenstand der Erfindung ist somit die Verwendung von Mehrschichtpigmenten auf Basis plättchenförmiger Substrate zur Einfärbung von Lebensmittel- und Pharmaerzeugnissen.

Im Vergleich zu den Produkten aus der DE 198 31 869 besitzen die Produkte, die mit einem Mehrschichtpigment gefärbt wurden, eine deutlich höhere Farbkraft und Transparenz, einen höheren Glanz und zeigen einen Farbflop zwischen zwei oder mehr Farben.

Die eingefärbten Lebensmittel und pharmazeutischen Erzeugnisse zeichnen sich durch einen Mehrfarbeneffekt aus, der beim Betrachter und Konsumenten angenehme subjektive Empfindungen hervorruft. Dieser optische Effekt ist mit den zur Zeit im Lebensmittelbereich zugelassenen Färbemitteln nicht möglich. Im Gegensatz zu Farbpigmenten, die im Lebensmittelbereich zugelassen sind, z. B. Pflanzenkohle E153, lassen sich die Mehrschichtpigmente auf Basis plättchenförmiger Substrate sehr leicht in das zu pigmentierende Medium dispergieren. Weiterhin zeichnen sich die so eingefärbten Erzeugnisse durch einen erhöhten Licht- und Feuchtigkeitsschutz aus. Insbesondere Vitaminpräparate sind länger haltbar. Bei der Einfärbung von Tabletten wurde in vielen Fällen eine verzögerte Abgabe von Wirkstoffen festgestellt.

Es zeigt sich, dass den Lebensmitteln bzw. Pharmaerzeugnissen bereits mit sehr geringen Mengen an Mehrschichtpigmenten neue interessante Farbtöne und gleichzeitig neue Eigenschaften verliehen werden können. Hervorragende Ergebnisse erzielt man schon bei der Einfärbung des Produkts mit 0,0025 bis 75,0 Gew.%, vorzugsweise 0,0025 bis 50 Gew.%, insbesondere 0,05 bis 25 Gew.%, an Mehrschichtpigment bezogen auf das Produkt.

In dem Fall, dass dem Lebensmittel- oder Pharmaerzeugnis das Pigment bei der Herstellung direkt beigemischt wird, beträgt die Einsatzmenge an Mehrschichtpigment vorzugsweise 0,005 bis 4 Gew.%. Bei der Oberflächenbehandlung von Lebensmitteln oder Tabletten liegt der Einsatzbereich bei 0,02 bis 15,0 Gew.%, vorzugsweise 0,5 bis 6,0 Gew.%, bezogen auf die Farb- bzw. Überzugslösung.

Geeignete Mehrschichtpigmente sind Pigmente auf Basis von mehrfach beschichteten Substraten, die sich dadurch auszeichnen, dass sie alternierend eine hoch- und eine niedrigbrechende Metalloxidschicht aufweisen.

Insbesondere geeignet sind Mehrschichtpigmente auf Basis von mehrfach beschichteten plättchenförmigen Substraten, die mindestens eine Schichtenfolge (A) (B) (A) enthalten, wobei
(A) eine hochbrechende Beschichtung bestehend aus Titandioxid und/oder Eisenoxid, und
(B) eine farblose niedrigbrechende Beschichtung mit einem Brechungsindex n ≤ 1,8
ist, wobei die Dicke aller Schichten auf dem Substrat ≤ 3 µm ist.

Geeignete Basissubstrate für die erfindungsgemäßen Mehrschichtpigmente sind transparente oder semitransparente plättchenförmige Substrate. Bevorzugte Substrate sind Schichtsilikate. Insbesondere geeignet sind natürlicher und/oder synthetischer Glimmer, Talkum, Kaolin, plättchenförmige Eisen- oder Aluminiumoxide, Glas-, SiO₂-, TiO₂-, plättchenförmige Mischoxide, wie z. B. FeTiO₃, Fe₂TiO₅, oder andere vergleichbare Materialien, abhängig von der jeweiligen gesetzlichen Zulässigkeit für die Verwendung bei Lebensmitteln bzw. pharmazeutischen Produkten.

Die Größe der Basissubstrate ist an sich nicht kritisch und kann auf den jeweiligen Anwendungszweck abgestimmt werden. In der Regel haben die plättchenförmigen Substrate eine Dicke zwischen 0,005 und 10 µm. insbesondere zwischen 0,05 und 5 µm. Die Ausdehnung in den beiden anderen Bereichen beträgt üblicherweise zwischen 1 und 500 µm, vorzugsweise zwischen 2 und 200 µm, und insbesondere zwischen 5 und 60 µm.

Die Dicke der einzelnen Schichten (A) und (B) mit hohem bzw. niedrigem Brechungsindex auf dem Basissubstrat ist wesentlich für die optischen Eigenschaften des Pigments. Für das Mehrschichtpigment mit intensivem Glanzeffekt muss die Dicke der einzelnen Schichten genau aufeinander eingestellt werden.

Die Dicke der Schicht (A) beträgt 10 - 500 nm, vorzugsweise 20 - 400 nm, insbesondere 30 - 350 nm. Die Dicke der Schicht (B) beträgt 10 - 500 nm, vorzugsweise 20 - 400 nm, insbesondere 30 - 350 nm.

Die Pigmente können mehrere, gleiche oder verschiedene Kombinationen an Schichtpaketen enthalten, bevorzugt ist aber die Belegung des Substrats mit nur einem Schichtpaket (A) (B) (A). Zur Intensivierung der Farbstärke kann das erfindungsgemäße Pigment bis zu 4 Schichtpakete enthalten, wobei die Dicke aller Schichten auf dem Substrat 3 µm allerdings nicht überschreiten sollte. Vorzugsweise wird eine ungerade Anzahl von Schichten auf das plättchenförmige Substrat aufgebracht mit je einer hochbrechenden Schicht in innerster und äußerster Lage. Besonders bevorzugt ist ein Aufbau von drei optischen Interferenzschichten in der Reihenfolge (A) (B) (A). Als hochbrechende Schicht kommen vorzugsweise TiO₂, Fe₂O₃ und/oder Fe₃O₄ in Frage. Das TiO₂ kann dabei in der Rutil-oder in der Anatasmodifikation vorliegen.

Als farblose niedrigbrechende für die Beschichtung (B) geeignete Materialien sind vorzugsweise Metalloxide bzw. die entsprechenden Oxidhydrate, wie z. B. SiO₂, Al₂O₃, AIO(OH), B₂O₃, MgF₂, MgSiO₃ oder ein Gemisch der genannten Metalloxide, geeignet entsprechend der gesetzlichen Zulassungen zur Verwendung bei Lebensmitteln bzw. pharmazeutischen Produkten. Bei der Schicht (B) handelt es sich insbesondere um eine SiO₂-Schicht.

Die bekannten Mehrschichtpigmente lassen sich leicht herstellen durch die Erzeugung mehrerer hoch- und niedrigbrechender Interferenzschichten mit genau definierter Dicke und glatter Oberfläche auf den feinteiligen, plättchenförmigen Substraten.

Die Metalloxidschichten werden vorzugsweise nasschemisch aufgebracht, wobei die zur Herstellung von Perlglanzpigmenten entwickelten nasschemischen Beschichtungsverfahren angewendet werden können. Derartige Verfahren sind z. B. beschrieben in DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017 oder auch in weiteren dem Fachmann bekannten Patentdokumenten und sonstigen Publikationen.

Weiterhin kann die Beschichtung auch in einem Wirbelbettreaktor durch Gasphasenbeschichtung erfolgen, wobei z. B. die in EP 0 045 851 und EP 0 106 235 zur Herstellung von Perlglanzpigmenten vorgeschlagenen Verfahren entsprechend angewendet werden können.

Der Farbton der Mehrschichtpigmente kann in sehr weiten Grenzen durch unterschiedliche Wahl der Belegungsmengen bzw. der daraus resultierenden Schichtdicken variiert werden. Die Feinabstimmung für einen bestimmten Farbton kann über die reine Mengenwahl hinaus durch visuell oder messtechnisch kontrolliertes Anfahren der gewünschten Farbe erreicht werden.

Besonders bevorzugte Mehrschichtpigmente besitzen folgenden Schichtaufbau:

| | | | | | | |
|---|---|---|---|---|---|---|
| Substrat | + | TiO₂ | + | SiO₂ | + | TiO₂ |
| Substrat | + | TiO₂ | + | SiO₂ | + | Fe₂O₃ |
| Substrat | + | TiO₂ | + | SiO₂ | + | Fe₃O₄ |
| Substrat | + | TiO₂ | + | Al₂O₃ | + | TiO₂ |
| Substrat | + | TiO₂ | + | Al₂O₃ | + | Fe₂O₃ |
| Substrat | + | TiO₂ | + | Al₂O₃ | + | Fe₂O₃ |
| Substrat | + | Fe₂O₃ | + | SiO₂ | + | TiO₂ |
| Substrat | + | Fe₃O₄ | + | SiO₂ | + | TiO₂ |
| Substrat | + | Fe₂O₃ | + | Al₂O₃ | + | TiO₂ |
| Substrat | + | Fe₃O₄ | + | Al₂O₃ | + | TiO₂ |

Insbesondere basieren die oben genannten Mehrschichtpigmente auf Glimmerplättchen, ferner auf Glas-, SiO₂- oder Al₂O₃-Plättchen.

Bei den verwendeten Mehrschichtpigmenten handelt es sich vorzugsweise um mit Magnetit und TiO₂ beschichtete natürliche oder synthetische Glimmerplättchen.

Durch die Zumischung von für den Lebensmittelbereich zugelassenen Perlglanzpigmenten, beschichteten oder unbeschichteten TiO₂, und/oder SiO₂-Plättchen, natürlichen bzw. naturidentischen Farbstoffen, organischen oder anorganischen Farbpigmenten oder färbenden natürlichen Frucht-und Pflanzenextrakten kann der Farbeffekt der Mehrschichtpigmente im Erzeugnis beeinflußt und gleichzeitig können neuartige changierende Farbeffekte erzielt werden.

Neben den Mehrschichtpigmenten können als weitere farbgebende Komponente alle dem Fachmann bekannten natürlichen oder naturidentischen Farbstoffe beigemischt werden. Insbesondere sind hier zu erwähnen E 101, E 104, E 110, E 124, E 131, E 132, E 140, E 141, E 151, E 160a. Weiterhin können auch andere Farbpigmente den plättchenförmigen Perlglanzpigmenten beigemischt werden, wie z.B. E 171, E 172, E 153.

Der Anteil an Farbstoffen bezogen auf das Erzeugnis liegt im Bereich von 0,5 bis 25 Gew.%. Als Farbstoff können ebenfalls Frucht- und Pflanzenextrakte eingesetzt werden, wie z.B. Möhrensaft, Rote Beete-Saft, Holundersaft, Hibiskussaft, Paprikaextrakt, Aroniaextrakt.

Durch die Kombination der Mehrschichtpigmente mit anderen Perlglanzpigmenten wie Gold-, Silber- oder Interferenzpigmenten wird der jeweilige Farbeffekt der Pigmente verstärkt. Dieser Synergismus erweitert die Farbmöglichkeiten der zu pigmentierenden Erzeugnisse erheblich, ohne dass andere natürliche oder naturidentische Farbstoffe zusätzlich eingesetzt werden müssen.

Die für den Lebensmittelbereich zugelassenen Mehrschichtpigmente sind im Handel erhältlich, beispielsweise unter der Marke Candurin^{®} ML von der Fa. Merck KGaA.

Die Gesamtkonzentration aller Pigmente im zu pigmentierenden Erzeugnis sollte ebenfalls 12 Gew.% bezogen auf das Erzeugnis nicht übersteigen. Sie ist in der Regel abhängig vom konkreten Anwendungsfall.

Die Einfärbung der Pharma- und Lebensmittelerzeugnisse erfolgt, indem das Mehrschichtpigment allein oder in Kombination mit weiteren Pigmenten oder Färbemitteln direkt oder in Gegenwart von Wasser und/oder eines organischen Lösungsmittels in den gewünschten Mengenverhältnissen, gleichzeitig oder nacheinander, während oder nach ihrer Herstellung dem einzufärbenden Erzeugnis zugegeben wird. Ein aufwendiges Mahlen und Dispergieren der Pigmente ist nicht erforderlich.

Bei der Einarbeitung in die Produktmatrix, z. B. Fruchtgummi, Getränke, etc. selbst, liegt die Einsatzmenge der Mehrschichtpigmente vorzugsweise bei 0,0025 - 10 Gew.%, insbesondere bei 0,05 - 3 Gew.%. Bei der Oberflächenfärbung von Lebensmitteln und Pharmaprodukten, z. B. Tabletten, Süßwaren, etc., liegt der Einsatzbereich in der verwendeten Farb- bzw. Überzugslösung bei 0,01 - 30 Gew.%, insbesondere bei 0,1 - 15 Gew. %. Bei der Verwendung der Mehrschichtpigmente in pulverförmigen Produkten liegt der Einsatzbereich bei 0,05 - 50 Gew. %, insbesondere bei 2 - 10 Gew.%.

Die Überzugslösungen enthalten vorzugsweise Wasser oder organische Lösemittel, vorzugsweise Ethanol oder Isopropanol. Als Filmbildner wird in den Überzugslösungen vorzugsweise ein Cellulosederivat, wie z.B. Hydroxypropylmethylcellulose, eingesetzt. Insbesondere bevorzugt sind Auftragslösungen mit Cellulosederivaten, die statt Wasser 5 - 80 Gew. % eines geeigneten organischen Lösemittels enthalten.

Gegenüber wässrigen Überzugslösungen besitzen die alkoholischen, bzw. alkoholisch-wäßrigen, Cellulose-haltigen Auftragslösungen deutliche anwendungstechnische Vorteile:
- Einsatz von kühlerer Trocknungsluft während des Sprühauftrags
- Färbung von wärmeempfindlichen Produkten, wie z. B. Schokoladefiguren, Lakritz, Schokoladedragees, etc. mit Mehrschichtpigmenten ist sehr gut möglich.

Als zur Einfärbung geeignete Produkte sind weiterhin insbesondere zu nennen Überzüge auf allen Arten von Lebensmitteln, insbesondere pigmentierte Zucker- und Schellacküberzüge (alkoholische und wässrige), Überzüge mit Ölen, Fetten, Stärken und Wachsen, mit Gummi Arabicum, mit Cellulosederivaten (z.B. HPMC = Hydroxypropylmethylcellulose), mit Stärke- und Eiweißderivaten, Carragenen und sonstigen dem Fachmann bekannten zum Überziehen geeigneten Substanzen, die Einarbeitung bzw. der Auftrag auf Zuckerwaren, Kuchendekorationen, Komprimate, Dragees, Kaugummis, Gummiwaren, Fondanterzeugnisse, Marzipanerzeugnisse, Füllmassen, Kakao- und Fettglasuren, Schokolade und schokoladehaltigen Produkten, Speiseeis, Cerealien, Snackprodukte, Überzugsmassen, Tortenspiegel, Zuckerstreusel, Nonpareilles, Gelee- und Gelatinewaren, Bonbons, Lakritze, Zuckerguss, Zuckerwatte, Fett-, Zucker- und Crememassen, Puddings, Desserts, Tortenguss, Kaltschalen, Lebensmitteln in Pulverform, Getränken, mit und ohne stabilisierenden Additiven wie z.B. Carboxymethylcellulose, gesäuerte und ungesäuerte Milchprodukte, wie z.B. Quark, Joghurt, Käse, Käserinden, Wursthüllen, etc.

Ein weiteres großes Einsatzgebiet liegt im Pharma- und OTC-Bereich zur Einfärbung von Tabletten, Hart- und Weich-Gelatinekapseln, Capsetten, Dragees, Salben, Hustensaft, Produkte in flüssiger und pulverförmiger Form, etc. In Kombination mit üblichen Coatings wie Polymethacrylaten und Cellulosearten, z.B. HPMC, können die Mehrschichtpigmente vielfältig zur Einfärbung eingesetzt werden.

Bei dragierten bzw. gecoateten Lebensmittel- und Pharmaerzeugnissen ist die Kombination der Mehrschichtpigmente mit Aromastoffen (Pulver- bzw. Flüssigaromen), Säuren und/oder mit Süßstoffen, wie z.B. Aspartam, möglich um den optischen Effekt auch geschmacklich zu betonen. Gegenstand der Erfindung sind somit alle Formulierungen aus dem Nahrungsmittel- und Pharmabereich enthaltend ein oder mehrere Mehrschichtpigmente allein oder in Kombination mit weiteren Pigmenten/Pigmentgemischen oder Farbstoffen (natürliche bzw. naturidentische) als Färbemittel.

Die nachfolgenden Beispiele sollen die Erfindung erläutern ohne sie jedoch zu begrenzen.

### Beispiele

### Beispiel 1: Fruchtgummiprodukte

Die Fruchtgummiprodukte werden wie gewohnt hergestellt. Die Verwendung von möglichst transparenten Gelbildnern verbessert den gewünschten Farbeffekt.

Die Mehrschichtpigmente werden in Wasser, Aromen und/oder Zucker vorsuspendiert. Dies verhindert die Bildung von unerwünschten und irreversiblen Pigmentagglomeraten.

### 1. Rezepturbeispiel

| **Komponente** | **Prozentualer Anteil** | **Bezugsquelle** |
|---|---|---|
| Wasser | 10,6945 % | |
| Zucker (Saccharose) | 31,45 % | Fa. Südzucker |
| Glukosesirup | 31,45 % | Fa. Cerestar, Krefeld |
| Candurin^{®} Blue ML** | 0,285 % (0,3 % bezogen auf die Gießmasse) | Fa. Merck KGaA |
| Zitronensäure 1:1 verd. | 2,51 % | Fa. Merck KGaA, |
| Gelatine (260 Bloom) | 7,86 % | Fa. DGF, Eberbach |
| Wasser | 15,748 % (Zum Auflösen der Gelatine) | |
| Farbstoff E129 | 0,0025 % | Fa. BASF |
| Aroma | Je nach gewünschter Geschmacksrichtung | |

| | | |
|---|---|---|
| ** Mehrschichtpigment auf Basis von Glimmer beschichtet mit TiO₂ und SiO₂ | | |

### 2. Rezepturbeispiel

| **Komponente** | **Prozentualer Anteil** | **Bezugsquelle** |
|---|---|---|
| Wasser | 10,598 % | |
| Zucker (Saccharose) | 31,45 % | Fa. Südzucker |
| Glukosesirup | 31,45 % | Fa. Cerestar, Krefeld |
| Candurin^{®} Yellow ML** | 0,38 % (0,4 % bezogen auf die Gießmasse) | Fa. Merck KGaA |
| Zitronensäure 1:1 verd. | 2,51 % | Fa. Merck KGaA |
| Gelatine (260 Bloom) | 7,86% | Fa. DGF, Eberbach |
| Wasser | 15,748 % (zum Auflösen der Gelatine) | |
| Farbstoff E133 | 0.0022 % | Fa. BASF |
| Farbstoff E102 | 0,0018 % | Fa. BASF |
| Aroma | Je nach gewünschter Geschmacksrichtung | |

| | | |
|---|---|---|
| ** Mehrschichtpigment auf Basis von Glimmer beschichtet mit TiO₂ und SiO₂ | | |

### Hersteltung:

Zunächst wird die Gelatine mit der doppelten Menge an Wasser bei 60 °C eingeweicht bzw. vorgequollen. Zucker und Wasser werden auf 100 °C erhitzt. Dann wird der Glukosesirup zugegeben. Man erhitzt weiter auf 120 °C und lässt das Ganze dann auf 85 °C abkühlen. Das Mehrschichtpigment, Zitronensäure, Aroma und die Gelatinelösung werden untergerührt. Die entlüftete Gießmasse wird mit dem Gießtrichter entweder in gefettete Formen oder in Stärkepuder gestempelte Negativformen abgefüllt. Das Produkt lässt man ca. 10-16 Stunden abkühlen. Die Fruchtgummiprodukte werden abschließend aus den Formen entnommen (beim Stärkepuder entpudert) und mit einem geeigneten Trennmittel behandelt.

Die Mehrschichtpigmente können aufgrund ihrer guten Temperatur- und pH-Stabilität zu jedem beliebigen Zeitpunkt zum Produktionsansatz gegeben werden.

### Beispiel 2: Dranierte Produkte

### 1. Beispiel

Zu färbendes Produkt: Grüne, zuckerdragierte Mandeln

Zusammensetzung der Auftrags-Sprühsuspension:

| **Komponente** | **Prozentualer Anteil** | **Bezugsquelle** |
|---|---|---|
| Candurin^{®} Blue ML | 3 % | Fa. Merck KGaA, |
| Sepifilm 050 | 5% | Fa. Seppic |
| Wasser | 92% | |

Auftragsmenge: Je nach gewünschter Farbintensität ca. 1-4 % Sprühsuspension/kg Produkt. Temperatur der Trocknungsluft: 35-45 °C

### 2. Beispiele

Zu färbendes Produkt: Braune, zuckerdragierte Haselnüsse mit Schokolade

Zusammensetzung der Auftrags-Sprühsuspension:

| **Komponente** | **Prozentualer Anteil** | **Bezugsquelle** |
|---|---|---|
| Candurin^{®} Gold ML** | 5 % | Fa. Merck KGaA |
| Sepifilm 050 | 5% | Fa. Seppic |
| Wasser | 50% | |
| Ethanol | 40% | Fa. Merck KGaA |

| | | |
|---|---|---|
| ** Mehrschichtpigment auf Basis von Glimmer beschichtet mit Fe₂O₃, SiO₂ und TiO₂ | | |

Auftragsmenge: Je nach gewünschter Farbintensität ca. 2-6 % Sprühsuspension/kg Produkt. Temperatur der Trocknungsluft: 28-35 °C

### 3. Beispiel

Zu färbendes Produkt: Mit dunkler Schokolade dragierte Erdnüsse

Zusammensetzung der Auftrags-Sprühsuspension:

| **Komponente** | **Prozentualer Anteil** | **Bezugsquelle** |
|---|---|---|
| Candurin^{®} Red ML** | 2 % | Fa. Merck KGaA |
| Sepifilm 050 | 5 % | Fa. Seppic |
| Wasser | 33 % | |
| Ethanol | 60 % | Fa. Merck KGaA |

| | | |
|---|---|---|
| ** Mehrschichtpigment auf Basis von Glimmer beschichtet mit TiO₂ und SiO₂ | | |

Auftragsmenge: Je nach gewünschter Farbintensität ca. 1-4 % Sprühsuspension/kg Produkt. Temperatur der Trocknungsluft: 26-28 °C

### 4. Beispiel

Zu färbendes Produkt: Rot dragierte Kaugummikugeln

Zusammensetzung der Auftrags-Sprühsuspension:

| **Komponente** | **Prozentualer Anteil** | **Bezugsquelle** |
|---|---|---|
| Candurin^{®} Yellow ML | 3 % | Fa. Merck KGaA |
| Sepifilm 050 | 5 % | Fa. Seppic |
| Wasser | 92% | |

Auftragsmenge: Je nach gewünschter Farbintensität 1-4 % Sprühsuspension/kg Produkt

Temperatur der Trocknungsluft: 35-45 °C

Herstellung der Sprühsuspension:

Die Mehrschichtpigmente werden in Wasser eingerührt bzw. suspendiert. Nun wird langsam der gewählte Filmbildner unter kontinuierlichem Rühren eingestreut. Je nach Temperaturbeständigkeit der zu färbenden Produkte kann nun auch Ethanol beigefügt werden.

Bedingt durch die ansteigende Viskosität der Suspension muss die Rührergeschwindigkeit dementsprechend angepasst werden. Je nach Zusammensetzung des Filmbildners ist dieser sofort in 20-60 Minuten vollständig gelöst. Die Suspension wird nun auf die Produkte aufgesprüht. Hierbei wird kontinuierlich entsprechende Trocknungsluft zugeführt. Es können je nach Wunsch noch Süßstoffe bzw. Aromen der Sprühsuspension zugefügt werden.

Der Auftrag kann in herkömmlichen, konventionellen Dragierkesseln, Dragierapparaturen bzw. in geschlossenen Film-Coatern durchgeführt werden. Als Film-Bildner eignen sich alle Materialien, welche die Pigmente in einem möglichst transparenten Film auf der Produktoberfläche fixieren (Cellulose-Arten, Carragenane, Schellack, etc.).

Der Sprühauftrag erfolgt in der Regel mit einer 2-Stoff-Sprühvorrichtung (airborne).

### Beispiel 3: Schokoladeartikel

### 1. Beispiel

### Färbung von Schokoladehohlfiguren (hergestellt aus dunkler, weißer und/oder Milchschokolade)

Zusammensetzung der Auftrags-Sprühsuspension:

| **Komponente** | **Prozentualer Anteil** | **Bezugsquelle** |
|---|---|---|
| Candurin^{®} Blue ML | 3 % | Fa. Merck KGaA |
| Capol 425 | 19,4% | Fa. Kaul GmbH |
| Ethanol | 77,6 % | Fa. Merck KGaA |

### 2. Beispiel

Färbung von Schaumartikel überzogen mit Schokolade

Zusammensetzung der Auftrags-Sprühsuspension:

| **Komponente** | **Prozentualer Anteil** | **Bezugsquelle** |
|---|---|---|
| Candurin^{®} Gold | 5 % | Fa. Merck KGaA |
| Capol 425 | 19% | Fa. Kaul GmbH |
| Ethanol | 76 % | Fa. Merck KGaA |

### 3. Beispiel

| **Komponente** | **Prozentualer Anteil** | **Bezugsquelle** |
|---|---|---|
| Candurin^{®} Green ML** | 3 % | Fa. Merck KGaA |
| Sepifilm 050 | 5 % | Fa. Seppic |
| Ethanol | 50 % | Fa. Merck KGaA |
| Wasser | 42 % | |

| | | |
|---|---|---|
| ** Mehrschichtpigment auf Basis von Glimmer beschichtet mit TiO₂ und SiO₂ | | |

Der Sprühauftrag kann mittels einer Sprühpistole (airborne) bzw. eines üblichen Airbrush erfolgen. Die Farbauftragsmenge ist abhängig vom gewünschten Farbeffekt.

Gerade die Verwendung eines hohen Ethanol-Anteils ermöglicht die Färbung solcher wärmeempfindlichen Lebensmittel, wie z.B. Schokoladeprodukte. Hierdurch bedingt kann die Verarbeitungstemperatur während des Färbevorganges niedrig gehalten werden. Die Produktqualität wird somit nicht beeinträchtigt.

### Beispiel 4: Getränke

| **Zutaten** | **Blaues Getränk mit rotem Glanzeffekt** | **Dunkelrotes Getränk mit goldenem Glanzeffekt** | **Bezugsquelle** |
|---|---|---|---|
| Candurin^{®} Red ML | 0.04% | Gold 0,05 % | Merck KGaA |
| Saccharose | 5% | 5% | - |
| Glukosesirup | 7% | 7% | - |
| Zitronensäure | 0,15% | 0.15% | Merck KGaA |
| Gelcarin(E407) Type GP-379NF | 0,2 % | 0,2% | FMC |
| Kelcogel^{®} (E418) | 0,0075 % | 0,0075 % | Kelco |
| Aroma | Nach Bedarf | Nach Bedarf | - |
| Zusätzlicher Farbstoff | E131* 0,0015 % | Provinol-Rotwein-Extrakt** 0,04% | *BASF ** Seppic Co. |
| Wasser | 87,601 % | 87,6025 % | - |

| | | | |
|---|---|---|---|
| pH-Wert > 3,5 | | | |

### Herstellung:

(1) Wasser vorlegen
(2) Candurin^{®} hinzufügen (in Wasser vorsuspendiert)
(3) Saccharose und Glucosesirup hinzufügen
(4) Verdicker hinzufügen (Gelcarin und Kelcarin^{®})
(5) Kräftig mixen
(6) auf 95-100 °C (1-2 Minuten) erhitzen, dabei kontinuierlich rühren
(7) unter kontinuierlichem Rühren auf ca. 40°C runterkühlen
(8) während der Abkühlphase Zugabe der Zitronensäure, zusätzliche Farbstoffe und Aromen
(9) Um eine Gelbildung zu vermeiden, sollte der abgekühlte Ansatz noch 30-45 Minuten gerührt werden.

Falls gewünscht, lassen sich gleiche Ergebnisse auch ohne die Zugabe entsprechender Verdickungsmittel erzielen. Bedingt durch die hohe Dichte der Pigmente setzen sich diese relativ schnell ab. Der Effekt kann dann durch kurzes Aufschütteln wieder hergestellt werden.

### Beispiel 5: Transparente Desserts (z.B. Wackelpudding)

| **Komponente** | **(%)** | **E-Nummer(n)** | **Bezugsquelle** | |
|---|---|---|---|---|
| Saccharose | 15% | - | - | A |
| Zitronensäure | 0,38 % | E330 | Merck KGaA | C |
| Calciumlactat | 0,05 % | E327 | Merck KGaA | A |
| tri-Kaliumphosphat | 0,05 % | E340iii | Merck KGaA | A |
| tetra-Natriumdiphosphat | 0,15% | E450iii | Merck KGaA | A |
| Genugel LC 4N* (Mischung von Carrageen und Johannisbrotkemmehl) | 0,6 % | E407 + E410 | Kelco | A |
| Kelcogel (Gellan gum) | 0,06 % | E418 | Kelco | A |
| Candurin^{®} Blue ML | 0,04 % | E171 + E555 | Merck KGaA | B |
| Zusätzlicher Farbstoff | 0,0015 % | E131 | BASF | C |
| Aroma | Nach Bedarf | - | - | C |
| Wasser | 83,6685 % | - | - | - |

### Herstellung:

(1) Wasser vorlegen
(2) Zufügen der pulverförmigen Zutaten (A)
(3) Gut vermischen und mit dem Erwärmen beginnen
(4) Zugabe von Candurin^{®} (vorsuspendiert in Wasser) (B)
(5) Erhitzung auf 95-100 °C (1-2 Minuten), kontinuierliches Rühren
(6) Abkühlphase
(7) Während der Abkühlphase Zugabe der Zitronensäure, zusätzlicher Farbstoffe und Aromen (C)
(8) Abkühlen auf 40-45 °C, kontinuierliches Rühren
(9) Masse in vorgesehene Gefäße abfüllen

### Beispiel 6: Tabletten

### Herstellung:

Die Färbung von Tabletten erfolgt mittels des sogenannten Film-Coating-Prozesses. Hierbei werden wässrige Auftragslösungen (Systeme mit Filmbildnern, Weichmachern, etc.) in sogenannten Coatern auf die darin rotierenden Tabletten kontinuierlich aufgesprüht.

### 1. Beispiel

Zu färbendes Produkt: Weiße Tabletten

Zusammensetzung der Coating-Lösung:

| **Komponente** | **Prozentualer Anteil** | **Bezugsquelle** |
|---|---|---|
| Candurin^{®} Gold ML | 5 % | Fa. Merck KGaA |
| Sepifilm 050 | 5% | Fa. Seppic |
| Wasser | 90% | |

Auftragsmenge: 15-20 g/kg Produkt

### 2. Beispiel

Zu färbendes Produkt: Weiße Tabletten

Zusammensetzung der Coating-Lösung:

| **Komponente** | **Prozentualer Anteil** | **Bezugsquelle** |
|---|---|---|
| Candurin^{®} Yellow ML | 3 % | Fa. Merck KGaA |
| Lustre Clear^{®} | 2,5 % | Fa. FMC |
| Wasser | 94,5 % | |

Auftragsmenge: 5-10 g/kg Produkt

### 3. Beispiel

Zu färbendes Produkt: Weiße Tabletten

Zusammensetzung der Coating-Lösung:

| **Komponente** | **Prozentualer Anteil** | **Bezugsquelle** |
|---|---|---|
| Candurin^{®} Blue ML | 5 % | Fa. Merck KGaA |
| Opagloss II | 6 % | Fa. Colorcon |
| Wasser | 89 % | |

Auftragsmenge: 5-10 g/kg Produkt

### 4. Beispiel

Zu färbendes Produkt: Weiße Tabletten

Zusammensetzung der Coating-Lösung:

| **Komponente** | **Prozentualer Anteil** | **Bezugsquelle** |
|---|---|---|
| Variochrom Magic Purple** | 5 % | Fa. BASF |
| Sepifilm 050 | 5 % | Fa. Seppic |
| Wasser | 90 % | |

| | | |
|---|---|---|
| ** Mehrschichtpigment auf Basis von Eisenoxid beschichtet mit SiO₂ und Eisenoxid | | |

Auftragsmenge: 20-25 g/kg Produkt

Die Menge der Auftragslösung hängt hierbei sowohl vom gewünschten Farbeffekt, als auch vom erforderlichen Polymerauftrag ab.

## Patentansprüche

1. Verwendung von Mehrschichtpigmenten auf Basis plättchenförmiger Substrate zur Einfärbung von Lebensmittel- oder Pharmaerzeugnissen, **dadurch gekennzeichnet, dass** das Mehrschichtpigment auf Basis von mehrfach beschichteten plättchenförmigen Substraten mindestens eine Schichtenfolge (A) (B) (A) enthält, wobei
(A) eine hochbrechende Beschichtung bestehend aus Titandioxid und/oder Eisenoxid, und
(B) eine farblose niedrigbrechende Beschichtung mit einem Brechungsindex n ≤ 1,8
ist,
wobei die Dicke aller Schichten auf dem Substrat ≤ 3 µm ist.

2. Verwendung von Mehrschichtpigmenten zur Einfärbung von Lebensmittel- oder Pharmaerzeugnissen nach Anspruch 1, **dadurch gekennzeichnet, dass** das plättchenförmige Substrat ein Glimmer-, Talkum-, Kaolin-, Aluminium-, Al₂O₃-, Fe₂O₃-, TiO₂-, Glas-, oder SiO₂-Plättchen ist.

3. Verwendung von Mehrschichtpigmenten zur Einfärbung von Lebensmittel- oder Pharmaerzeugnissen nach Anspruch 1 oder 2, **dadurch gekennzeichnet, dass** das plättchenförmige Substrat natürlicher und/oder synthetischer Glimmer ist.

4. Verwendung von Mehrschichtpigmenten zur Einfärbung von Lebensmittel- oder Pharmaerzeugnissen nach einem oder mehreren der Ansprüche 1 bis 3, **dadurch gekennzeichnet, dass** die hochbrechende Schicht (A) TiO₂, Fe₂O₃ und/oder Fe₃O₄ ist.

5. Verwendung von Mehrschichtpigmenten zur Einfärbung von Lebensmittel- oder Pharmaerzeugnissen nach einem oder mehreren der Ansprüche 1 bis 4, **dadurch gekennzeichnet, dass** die niedrigbrechende Schicht (B) SiO₂, Al₂O₃, AlO(OH), B₂O₃, MgF₂, MgSiO₃ oder ein Gemisch der genannten Metalloxide ist.

6. Verwendung von Mehrschichtpigmenten zur Einfärbung von Lebensmittel- oder Pharmaerzeugnissen nach einem oder mehreren der Ansprüche 1 bis 5, **dadurch gekennzeichnet, dass** die Schicht (A) des Mehrschichtpigments eine Dicke von 10-500 nm aufweist.

7. Verwendung von Mehrschichtpigmenten zur Einfärbung von Lebensmittel- oder Pharmaerzeugnissen nach einem oder mehreren der Ansprüche 1 bis 6, **dadurch gekennzeichnet, dass** die Schicht (B) des Mehrschichtpigments eine Dicke von 10-500 nm aufweist.

8. Verwendung von Mehrschichtpigmenten zur Einfärbung von Lebensmittel- oder Pharmaerzeugnissen nach einem oder mehreren der Ansprüche 1 bis 7, **dadurch gekennzeichnet, dass** Mehrschichtpigment nur ein Schichtpaket (A)(B)(A) auf dem Substrat aufweist.

9. Verwendung von Mehrschichtpigmenten zur Einfärbung von Lebensmittel- oder Pharmaerzeugnissen nach einem oder mehreren der Ansprüche 1 bis 8, **dadurch gekennzeichnet, dass** das Mehrschichtpigment folgenden Schichtaufbau besitzt:
| | | | | | | |
|---|---|---|---|---|---|---|
| Substrat | + | TiO₂ | + | SiO₂ | + | TiO₂ |
| Substrat | + | TiO₂ | + | SiO₂ | + | Fe₂O₃ |
| Substrat | + | TiO₂ | + | SiO₂ | + | Fe₃O₄ |
| Substrat | + | TiO₂ | + | Al₂O₃ | + | TiO₂ |
| Substrat | + | TiO₂ | + | Al₂O₃ | + | Fe₂O₃ |
| Substrat | + | TiO₂ | + | Al₂O₃ | + | Fe₂O₃ |
| Substrat | + | Fe₂O₃ | + | SiO₂ | + | TiO₂ |
| Substrat | + | Fe₃O₄ | + | SiO₂ | + | TiO₂ |
| Substrat | + | Fe₂O₃ | + | Al₂O₃ | + | TiO₂ |
| Substrat | + | Fe₃O₄ | + | Al₂O₃ | + | TiO₂ |

10. Verwendung von Mehrschichtpigmenten zur Einfärbung von Lebensmittel- oder Pharmaerzeugnissen nach einem oder mehreren der Ansprüche 1 bis 9, **dadurch gekennzeichnet, dass** das TiO₂ im Mehrschichtpigment in der Rutil- oder in der Anatasmodifikation vorliegt.

11. Verwendung von Mehrschichtpigmenten zur Einfärbung von Lebensmittel- oder Pharmaerzeugnissen nach einem oder mehreren der Ansprüche 1 bis 10, **dadurch gekennzeichnet, dass** der Anteil an Mehrschichtpigment im Lebensmittel- oder Pharmaerzeugnis 0,0025 bis 75 Gew. % bezogen auf das Produkt beträgt.

12. Verwendung von Mehrschichtpigmenten zur Einfärbung von Lebensmittel- oder Pharmaerzeugnissen nach einem oder mehreren der Ansprüche 1 bis 11, **dadurch gekennzeichnet, dass** der Anteil an Mehrschichtpigment im Lebensmittel- oder Pharmaerzeugnis 0,005 bis 15 Gew. % beträgt.

13. Verwendung von Mehrschichtpigmenten zur Einfärbung von Lebensmittel- oder Pharmaerzeugnissen nach einem oder mehreren der Ansprüche 1 bis 12, **dadurch gekennzeichnet, dass** die Mehrschichtpigmente in Kombination mit ein oder mehreren Perlglanzpigmenten, beschichteten oder unbeschichteten TiO₂-Plättchen, SiO₂-Plättchen, natürlichen bzw. naturidentischen Farbstoffen, Farbpigmenten oder natürlichen färbenden Pflanzen-oder Fruchtextrakten eingesetzt werden.

14. Verwendung von Mehrschichtpigmenten zur Einfärbung von Lebensmittel- oder Pharmaerzeugnissen nach einem oder mehreren der Ansprüche 1 bis 13, **dadurch gekennzeichnet, dass** die Mehrschichtpigmente mit natürlichen oder naturidentischen Farbstoffen beigemischt werden.

15. Verwendung von Mehrschichtpigmenten zur Einfärbung von Lebensmittel- oder Pharmaerzeugnissen nach einem der Ansprüche 1 bis 14, **dadurch gekennzeichnet, dass** die Mehrschichtpigmente mit E 101, E 104, E 110, E 124, E 131, E 132, E 140, E 141, E 151, E160a beigemischt werden.

16. Verwendung von Mehrschichtpigmenten zur Einfärbung von Lebensmittel- oder Pharmaerzeugnissen nach einem oder mehreren der Ansprüche 1 bis 15, **dadurch gekennzeichnet, dass** die Mehrschichtpigmente mit Farbpigmenten beigemischt werden.

17. Verwendung von Mehrschichtpigmenten zur Einfärbung von Lebensmittel- oder Pharmaerzeugnissen nach einem oder mehreren der Ansprüche 1 bis 16, **dadurch gekennzeichnet, dass** die Mehrschichtpigmente mit E 171, E 172, E 153 beigemischt werden.

18. Verwendung von Mehrschichtpigmenten zur Einfärbung von Lebensmittel- oder Pharmaerzeugnissen nach einem oder mehreren der Ansprüche 1 bis 17, **dadurch gekennzeichnet, dass** die Mehrschichtpigmente in Kombination mit Frucht- und Pflanzenextrakten eingesetzt werden.

19. Verwendung von Mehrschichtpigmenten zur Einfärbung von Lebensmittel- oder Pharmaerzeugnissen nach einem oder mehreren der Ansprüche 1 bis 18, **dadurch gekennzeichnet, dass** die Mehrschichtpigmente in Kombination mit Möhrensaft, Rote Beete-Saft, Holundersaft, Hibiskussaft, Paprikaextrakt, Aroniaextrakt eingesetzt werden.

20. Verwendung von Mehrschichtpigmenten zur Einfärbung von dragierten bzw. gecoateten Lebensmittel- oder Pharmaerzeugnissen nach einem oder mehreren der Ansprüche 1 bis 19, **dadurch gekennzeichnet, dass** die Mehrschichtpigmente in Kombination mit Aromastoffen, Säuren und/oder Süßstoffen eingesetzt werden.

21. Verwendung von Mehrschichtpigmenten zur Einfärbung von Lebensmittel- oder Pharmaerzeugnissen nach einem oder mehreren der Ansprüche 1 bis 20, **dadurch gekennzeichnet, dass** das Lebensmittel- oder Pharmaerzeugnis mit einem Überzug aus Cellulosederivaten, Schellack, Ölen, Wachsen, Gummi Arabicum, Cellulosearten, Polymethacrylaten, Stärke- oder Eiweißbasis, Stärke-oder Eiweißderivate, Fetten und Fettderivaten oder Zuckerguss enthaltend Mehrschichtpigmente und gegebenenfalls weiteren Pigmenten und/oder Färbemitteln versehen ist.

22. Verfahren zur Herstellung von mit Mehrschichtpigmenten eingefärbten Lebensmittel- und Pharmaerzeugnissen nach einem oder mehreren der Ansprüche 1 bis 21, **dadurch gekennzeichnet, dass** das Mehrschichtpigment allein oder in Kombination mit weiteren Pigmenten oder Färbemitteln direkt oder in Gegenwart von Wasser und/oder eines organischen Lösungsmittels in den gewünschten Mengenverhältnissen, gleichzeitig oder nacheinander, während oder nach ihrer Herstellung dem einzufärbenden Erzeugnis zugegeben wird.

23. Lebensmittel- und Pharmaerzeugnisse enthaltend Mehrschichtpigmente auf Basis plättchenförmiger Substrate nach einem oder mehreren der Ansprüche 1 bis 21 als Färbemittel.

## Claims

1. Use of multilayered pigments based on flake-form substrates for colouring food or pharmaceutical products, **characterised in that** the multilayered pigment based on multicoated flake-form substrates comprises at least one layer sequence (A) (B) (A), where
(A) is a high-refractive-index coating consisting of titanium dioxide and/or iron oxide, and
(B) is a colourless, low-refractive-index coating having a refractive index n ≤ 1.8,
where the thickness of all layers on the substrate is ≤ 3 µm.

2. Use of multilayered pigments for colouring food or pharmaceutical products according to Claim 1, **characterised in that** the flake-form substrate is a mica, talc, kaolin, aluminium, Al₂O₃, Fe₂O₃, TiO₂, glass or SiO₂ flake.

3. Use of multilayered pigments for colouring food or pharmaceutical products according to Claim 1 or 2, **characterised in that** the flake-form substrate is natural and/or synthetic mica.

4. Use of multilayered pigments for colouring food or pharmaceutical products according to one or more of Claims 1 to 3, **characterised in that** the high-refractive-index layer (A) is TiO₂, Fe₂O₃ and/or Fe₃O₄.

5. Use of multilayered pigments for colouring food or pharmaceutical products according to one or more of Claims 1 to 4, **characterised in that** the low-refractive-index layer (B) is SiO₂, Al₂O₃, AIO(OH), B₂O₃, MgF₂, MgSiO₃ or a mixture of the said metal oxides.

6. Use of multilayered pigments for colouring food or pharmaceutical products according to one or more of Claims 1 to 5, **characterised in that** layer (A) of the multilayered pigment has a thickness of 10-500 nm.

7. Use of multilayered pigments for colouring food or pharmaceutical products according to one or more of Claims 1 to 6, **characterised in that** layer (B) of the multilayered pigment has a thickness of 10-500 nm.

8. Use of multilayered pigments for colouring food or pharmaceutical products according to one or more of Claims 1 to 7, **characterised in that** the multilayered pigment has only one layer package (A)(B)(A) on the substrate.

9. Use of multilayered pigments for colouring food or pharmaceutical products according to one or more of Claims 1 to 8, **characterised in that** the multilayered pigment has the following layer structure:
| | | | | | | |
|---|---|---|---|---|---|---|
| Substrat | + | TiO₂ | + | SiO₂ | + | TiO₂ |
| Substrat | + | TiO₂ | + | SiO₂ | + | Fe₂O₃ |
| Substrat | + | TiO₂ | + | SiO₂ | + | Fe₃O₄ |
| Substrat | + | TiO₂ | + | Al₂O₃ | + | TiO₂ |
| Substrat | + | TiO₂ | + | Al₂O₃ | + | Fe₂O₃ |
| Substrat | + | TiO₂ | + | Al₂O₃ | + | Fe₂O₃ |
| Substrat | + | Fe₂O₃ | + | SiO₂ | + | TiO₂ |
| Substrat | + | Fe₃O₄ | + | SiO₂ | + | TiO₂ |
| Substrat | + | Fe₂O₃ | + | Al₂O₃ | + | TiO₂ |
| Substrat | + | Fe₃O₄ | + | Al₂O₃ | + | TiO₂ |

10. Use of multilayered pigments for colouring food or pharmaceutical products according to one or more of Claims 1 to 9, **characterised in that** the TiO₂ in the multilayered pigment is in the rutile or anatase modification.

11. Use of multilayered pigments for colouring food or pharmaceutical products according to one or more of Claims 1 to 10, **characterised in that** the proportion of multilayered pigment in the food or pharmaceutical product is 0.0025 to 75% by weight, based on the product.

12. Use of multilayered pigments for colouring food or pharmaceutical products according to one or more of Claims 1 to 11, **characterised in that** the proportion of multilayered pigment in the food or pharmaceutical product is 0.005 to 15% by weight.

13. Use of multilayered pigments for colouring food or pharmaceutical products according to one or more of Claims 1 to 12, **characterised in that** the multilayered pigments are employed in combination with one or more pearlescent pigments, coated or uncoated TiO₂ flakes, SiO₂ flakes, natural or nature-identical dyes, coloured pigments or natural colouring plant or fruit extracts.

14. Use of multilayered pigments for colouring food or pharmaceutical products according to one or more of Claims 1 to 13, **characterised in that** the multilayered pigments are admixed with natural or nature-identical dyes.

15. Use of multilayered pigments for colouring food or pharmaceutical products according to one of Claims 1 to 14, **characterised in that** the multilayered pigments are admixed with E 101, E 104, E 110, E 124, E 131, E 132, E 140, E 141, E 151, E 160a.

16. Use of multilayered pigments for colouring food or pharmaceutical products according to one or more of Claims 1 to 15, **characterised in that** the multilayered pigments are admixed with coloured pigments.

17. Use of multilayered pigments for colouring food or pharmaceutical products according to one or more of Claims 1 to 16, **characterised in that** the multilayered pigments are admixed with E 171, E 172, E 153.

18. Use of multilayered pigments for colouring food or pharmaceutical products according to one or more of Claims 1 to 17, **characterised in that** the multilayered pigments are employed in combination with fruit and plant extracts.

19. Use of multilayered pigments for colouring food or pharmaceutical products according to one or more of Claims 1 to 18, **characterised in that** the multilayered pigments are employed in combination with carrot juice, beetroot juice, elderberry juice, hibiscus juice, paprika extract, aronia extract.

20. Use of multilayered pigments for colouring coated food or pharmaceutical products according to one or more of Claims 1 to 19, **characterised in that** the multilayered pigments are employed in combination with aroma substances, acids and/or sweeteners.

21. Use of multilayered pigments for colouring food or pharmaceutical products according to one or more of Claims 1 to 20, **characterised in that** the food or pharmaceutical product is provided with a coating of cellulose derivatives, shellac, oils, waxes, gum arabic, cellulose products, polymethacrylates, starch or albumen base, starch or albumen derivatives, fats and fat derivatives or icing comprising multilayered pigments and optionally further pigments and/or colorants.

22. Process for the production of food and pharmaceutical products coloured with multilayered pigments according to one or more of Claims 1 to 21, **characterised in that** the multilayered pigment, alone or in combination with further pigments or colorants, is added to the product to be coloured, directly or in the presence of water and/or an organic solvent in the desired mixing ratios, simultaneously or successively, during or after production thereof.

23. Food and pharmaceutical products comprising multilayered pigments based on flake-form substrates according to one or more of Claims 1 to 21 as colorants.

## Revendications

1. Utilisation de pigments multicouches à base de substrats sous forme de paillettes pour la coloration de produits alimentaires ou pharmaceutiques, **caractérisée en ce que** le pigment multicouches à base de substrats sous forme de paillettes à revêtements multiples comprend au moins une séquence de couches (A) (B) (A), où
(A) est un revêtement à indice de réfraction élevé constitué de dioxyde de titane et/ou oxyde de fer, et
(B) est un revêtement incolore à indice de réfraction faible ayant un indice de réfraction n ≤ 1,8,
où l'épaisseur de toutes les couches sur le substrat est ≤ 3 µm.

2. Utilisation de pigments multicouches pour la coloration de produits alimentaires ou pharmaceutiques selon la revendication 1, **caractérisée en ce que** le substrat sous forme de paillettes est une paillette de mica, talc, kaolin, aluminium, Al₂O₃, Fe₂O₃, TiO₂, verre ou SiO₂.

3. Utilisation de pigments multicouches pour la coloration de produits alimentaires ou pharmaceutiques selon la revendication 1 ou 2, **caractérisée en ce que** le substrat sous forme de paillettes est du mica naturel et/ou synthétique.

4. Utilisation de pigments multicouches pour la coloration de produits alimentaires ou pharmaceutiques selon l'une ou plusieurs des revendications 1 à 3, **caractérisée en ce que** la couche à indice de réfraction élevé (A) est TiO₂, Fe₂O₃ et/ou Fe₃O₄.

5. Utilisation de pigments multicouches pour la coloration de produits alimentaires ou pharmaceutiques selon l'une ou plusieurs des revendications 1 à 4, **caractérisée en ce que** la couche à indice de réfraction faible (B) est SiO₂, Al₂O₃, AlO(OH), B₂O₃, MgF₂, MgSiO₃ ou un mélange desdits oxydes métalliques.

6. Utilisation de pigments multicouches pour la coloration de produits alimentaires ou pharmaceutiques selon l'une ou plusieurs des revendications 1 à 5, **caractérisée en ce que** la couche (A) du pigment multicouches a une épaisseur de 10-500 nm.

7. Utilisation de pigments multicouches pour la coloration de produits alimentaires ou pharmaceutiques selon l'une ou plusieurs des revendications 1 à 6, **caractérisée en ce que** la couche (B) du pigment multicouches a une épaisseur de 10-500 nm.

8. Utilisation de pigments multicouches pour la coloration de produits alimentaires ou pharmaceutiques selon l'une ou plusieurs des revendications 1 à 7, **caractérisée en ce que** le pigment multicouches ne possède qu'un ensemble de couches (A)(B)(A) sur le substrat.

9. Utilisation de pigments multicouches pour la coloration de produits alimentaires ou pharmaceutiques selon l'une ou plusieurs des revendications 1 à 8, **caractérisée en ce que** le pigment multicouches possède la structure de couches suivante :
| | | | | | | |
|---|---|---|---|---|---|---|
| Substrat | + | TiO₂ | + | SiO₂ | + | TiO₂ |
| Substrat | + | TiO₂ | + | SiO₂ | + | Fe₂O₃ |
| Substrat | + | TiO₂ | + | SiO₂ | + | Fe₃O₄ |
| Substrat | + | TiO₂ | + | Al₂O₃ | + | TiO₂ |
| Substrat | + | TiO₂ | + | Al₂O₃ | + | Fe₂O₃ |
| Substrat | + | TiO₂ | + | Al₂O₃ | + | Fe₂O₃ |
| Substrat | + | Fe₂O₃ | + | SiO₂ | + | TiO₂ |
| Substrat | + | Fe₃O₄ | + | SiO₂ | + | TiO₂ |
| Substrat | + | Fe₂O₃ | + | Al₂O₃ | + | TiO₂ |
| Substrat | + | Fe₃O₄ | + | Al₂O₃ | + | TiO₂ |

10. Utilisation de pigments multicouches pour la coloration de produits alimentaires ou pharmaceutiques selon l'une ou plusieurs des revendications 1 à 9, **caractérisée en ce que** le TiO₂ dans le pigment multicouches est selon la modification de rutile ou d'anatase.

11. Utilisation de pigments multicouches pour la coloration de produits alimentaires ou pharmaceutiques selon l'une ou plusieurs des revendications 1 à 10, **caractérisée en ce que** la proportion de pigment multicouches dans le produit alimentaire ou pharmaceutique va de 0,0025 à 75% en poids, sur la base du produit.

12. Utilisation de pigments multicouches pour la coloration de produits alimentaires ou pharmaceutiques selon l'une ou plusieurs des revendications 1 à 11, **caractérisée en ce que** la proportion de pigment multicouches dans le produit alimentaire ou pharmaceutique va de 0,005 à 15% en poids.

13. Utilisation de pigments multicouches pour la coloration de produits alimentaires ou pharmaceutiques selon l'une ou plusieurs des revendications 1 à 12, **caractérisée en ce que** les pigments multicouches sont employés en combinaison avec un(e) ou plusieurs pigments nacrés, paillettes de TiO₂ revêtues ou non revêtues, paillettes de SiO₂, colorants naturels ou identiques aux colorants naturels, pigments colorés ou extraits de plantes ou de fruits colorants naturels.

14. Utilisation de pigments multicouches pour la coloration de produits alimentaires ou pharmaceutiques selon l'une ou plusieurs des revendications 1 à 13, **caractérisée en ce que** les pigments multicouches sont mélangés avec des colorants naturels ou identiques aux colorants naturels.

15. Utilisation de pigments multicouches pour la coloration de produits alimentaires ou pharmaceutiques selon l'une des revendications 1 à 14, **caractérisée en ce que** les pigments multicouches sont mélangés avec du E 101, E 104, E 110, E 124, E 131, E 132, E 140, E 141, E 151, E 160a.

16. Utilisation de pigments multicouches pour la coloration de produits alimentaires ou pharmaceutiques selon l'une ou plusieurs des revendications 1 à 15, **caractérisée en ce que** les pigments multicouches sont mélangés avec des pigments colorés.

17. Utilisation de pigments multicouches pour la coloration de produits alimentaires ou pharmaceutiques selon l'une ou plusieurs des revendications 1 à 16, **caractérisée en ce que** les pigments multicouches sont mélangés avec du E 171, E 172, E 153.

18. Utilisation de pigments multicouches pour la coloration de produits alimentaires ou pharmaceutiques selon l'une ou plusieurs des revendications 1 à 17, **caractérisée en ce que** les pigments multicouches sont employés en combinaison avec des extraits de plantes et de fruits.

19. Utilisation de pigments multicouches pour la coloration de produits alimentaires ou pharmaceutiques selon l'une ou plusieurs des revendications 1 à 18, **caractérisée en ce que** les pigments multicouches sont employés en combinaison avec du jus de carotte, du jus de betterave, du jus de sureau, du jus d'hibiscus, de l'extrait de paprika, de l'extrait d'aronia.

20. Utilisation de pigments multicouches pour la coloration de produits alimentaires ou pharmaceutiques enrobés selon l'une ou plusieurs des revendications 1 à 19, **caractérisée en ce que** les pigments multicouches sont employés en combinaison avec des substances aromatiques, des acides et/ou des édulcorants.

21. Utilisation de pigments multicouches pour la coloration de produits alimentaires ou pharmaceutiques selon l'une ou plusieurs des revendications 1 à 20, **caractérisée en ce que** le produit alimentaire ou pharmaceutique est pourvu d'un enrobage de dérivés de cellulose, de gomme laque, d'huiles, de cires, de gomme arabique, de produits de cellulose, de polyméthacrylates, à base d'amidon ou d'albumen, de dérivés d'amidon ou d'albumen, de matières grasses et de dérivés de matières grasses ou de glaçage comprenant des pigments multicouches et éventuellement d'autres pigments et/ou colorants.

22. Procédé de production de produits alimentaires et pharmaceutiques colorés par des pigments multicouches selon l'une ou plusieurs des revendications 1 à 21, **caractérisé en ce que** le pigment multicouches, seul ou en combinaison avec d'autres pigments ou colorants, est ajouté au produit à colorer, directement ou en présence d'eau et/ou d'un solvant organique selon les rapports de mélange souhaités, simultanément ou successivement, pendant ou après la production de ceux-ci.

23. Produits alimentaires et pharmaceutiques comprenant des pigments multicouches à base de substrats sous forme de paillettes selon l'une ou plusieurs des revendications 1 à 21, comme colorants.
